# FASCICULE DE BREVET EUROPEEN

(11) **EP 2 285 344 B1**
(45) Date de publication et mention de la délivrance du brevet: **18.04.2018**
(21) Numéro de dépôt: 09757716.7
(22) Date de dépôt: 11.05.2009
(51) Int. Cl.: A61K 8/99, A61Q 11/00, A61K 36/05, A61K 8/97, A23G 3/48, A23G 3/38, A61P 1/02, A23G 3/00

(54) **CONFISERIE AUX ALGUES POUR LA PREVENTION DES INFECTIONS BUCCODENTAIRES**
SÜSSSTOFFHALTIGE ALGEN ZUR VERHINDERUNG VON ZAHN- UND MUNDINFEKTIONEN
SWEET CONTAINING ALGAE FOR THE PREVENTION OF ORO-DENTAL INFECTIONS

(30) Priorité: 14.05.2008 FR 0853128
(43) Date de publication de la demande: 23.02.2011
(73) Titulaire: Roquette Frères, 62136 Lestrem (FR)
(72) Inventeur: RIBADEAU-DUMAS, Guillaume, F-59237 Verlinghem (FR)
(74) Mandataire: Cabinet Plasseraud
(86) Numéro de dépôt international: PCT/FR2009/050858
(87) Numéro de publication internationale: WO 2009/147340

(56) Documents cités:
- WO-A-01/26617
- WO-A1-02/34279
- WO-A1-99/44573
- JP-A- H05 139 979
- JP-B2- 3 113 548
- US-A1- 2004 081 665
- US-A1- 2005 266 018
- DATABASE WPI Week 200234 Thomson Scientific, London, GB; AN 2002-295746 XP002508903 & JP 2001 240604 A (LION CORP) 4 septembre 2001 (2001-09-04)
- DATABASE WPI Week 199039 Thomson Scientific, London, GB; AN 1990-296105 XP002508906 & SU 1 528 495 A (KRASD SOUVENIR WKS) 15 décembre 1989 (1989-12-15)
- DATABASE WPI Week 198818 Thomson Scientific, London, GB; AN 1988-121852 XP002508904 & HU 44 432 A (SZATLOCZKY E) 28 mars 1988 (1988-03-28)
- DATABASE WPI Week 199717 Thomson Scientific, London, GB; AN 1997-188290 XP002508905 & JP 09 048715 A (LION CORP) 18 février 1997 (1997-02-18)
- DATABASE WPI Week 200431 Thomson Scientific, London, GB; AN 2004-336549 XP002508909 & JP 2004 123578 A (BAIHORON KK) 22 avril 2004 (2004-04-22)
- DATABASE WPI Week 199715 Thomson Scientific, London, GB; AN 1997-159072 XP002508907 & JP 09 028356 A (MICRO ALGE CORP KK) 4 février 1997 (1997-02-04)

## Description

### DOMAINE DE L'INVENTION

La présente invention concerne de façon générale une confiserie. Plus spécifiquement, la présente invention concerne une confiserie visant à procurer des bienfaits dentaires. La confiserie selon la présente invention favorise une bonne hygiène bucco-dentaire, et de manière plus particulière, favorise et maintient les dents et les gencives saines. Elle se caractérise en ce qu'elle contient des microalgues et plus particulièrement de la chlorelle.

### ARRIERE-PLAN TECHNOLOGIQUE

Les algues font partie des premiers organismes apparus sur Terre, et sont définies comme des organismes eucaryotes dépourvus de racines, de tige et de feuille, mais possédant de la chlorophylle ainsi que d'autres pigments accessoires à la photosynthèse productrice d'oxygène. Elles sont bleues, rouges, jaunes, doré et brun ou encore vertes. Elles représentent plus de 90% des végétaux marins et 18% du règne végétal, avec leurs 40 000 à 45 000 espèces. Les algues sont des organismes extrêmement variés tant par leur taille et leur forme que par leur structure cellulaire. Elles vivent en milieu aquatique ou très humide. Elles contiennent de nombreuses vitamines et oligo-éléments, et sont de véritables concentrés d'actifs stimulants et bienfaisants pour la santé et la beauté. Elles ont des vertus anti-inflammatoires, hydratantes, adoucissantes, régénérantes, raffermissantes, anti-âge. Elles possèdent également des caractéristiques "technologiques" qui permettent d'apporter de la texture à un produit alimentaire. En effet, les fameux additifs E400 à E407 ne sont en fait que des composés extraits d'algues, dont on utilise les propriétés épaississantes, gélifiantes, émulsifiantes et stabilisantes.

Les microalgues au sens strict sont des algues microscopiques. Unicellulaires ou pluricellulaires indifférenciées, ce sont des micro-organismes photosynthétiques séparés en deux groupes polyphylétiques : les eucaryotes et les procaryotes. Vivant dans les milieux fortement aqueux, elles peuvent posséder une mobilité flagellaire.

La chlorelle (ou chlorella) est une algue unicellulaire microscopique d'eau douce apparue sur la terre depuis plus de 3 milliards d'années. Elle fut découverte en 1890 par un microbiologiste hollandais Martinus Beijernick, fasciné par son contenu en chlorophylle et par la présence d'un élément lui conférant un taux de multiplication élevé : le CGF (facteur de croissance chlorelle). La chlorelle possède la plus grande concentration en chlorophylle de tous les végétaux, et sa capacité à la photosynthèse est considérable. Depuis sa découverte, la chlorelle n'a cessé de générer un intérêt considérable dans le monde, et aujourd'hui elle est produite à grande échelle pour des utilisations dans des compléments alimentaires et nutritionnels. En effet, la chlorelle contient plus de 60% de protéines qui renferment beaucoup d'acides aminés essentiels au bien-être humain et animal. La chlorelle contient également beaucoup de vitamines (A, Bêta carotène, B1 : thiamine, B2 : riboflavine, B3 : niacine, B5 : acide panthoténique, B6 : pyridoxine, B9 : acide folique, B12 : cobalamine, vitamine C : acide ascorbique, vitamine E : tocophérol, vitamine K : phylloquinone), de lutéine (famille des caroténoïdes, puissant antioxydant), et de minéraux dont le calcium, le fer, le phosphore, le manganèse, le potassium, le cuivre et le zinc. En outre, la chlorelle contient certains acides gras polyinsaturés de type oméga indispensables au bon fonctionnement cardiaque et cérébral et à la prévention de nombreuses maladies comme le cancer, le diabète ou l'obésité. La chlorelle dispose de trois éléments participant à son action :
- la chlorophylle présente dans la chlorelle à plus de 4% (en poids) et connue pour son action purifiante.
- La membrane cellulaire : non digérée par le tube digestif humain dépourvu des enzymes nécessaires à la dégradation de la cellulose, elle fixe les métaux lourds et les toxines et en accélère l'élimination par les voies naturelles.
- Le C.F.G présent à hauteur de 5% (en poids) est un puissant tonifiant du métabolisme et de la croissance cellulaires. C'est un élément inestimable provenant du noyau de la cellule, et contenant essentiellement des acides aminés, des beta-glucanes et des acides nucléiques. Il confère à la chlorelle un taux de division, de multiplication et de croissance cellulaire très rapide.

Les bienfaits rapportés à la consommation de chlorelle sont très nombreux. C'est un complément alimentaire utilisé quotidiennement au Japon par 4 millions de personnes. A tel point que le gouvernement japonais l'a classée comme « aliment d'intérêt national ». Cette algue précieuse est principalement, mais pas uniquement conseillée pour :
- apaiser le système nerveux et favoriser le sommeil ;
- réduire la constipation, normaliser le transit même chez les paralysés. Elle restaure également la flore intestinale ;
- stimuler le système immunitaire ;
- favoriser l'équilibre acido-basique ;
- stimuler la régénération cellulaire et ralentir le vieillissement ;
- diminuer les graisses dans le sang et réduire le risque des maladies cardio-vasculaires ;
- réduire une hypertension ;
- fixer les métaux lourds et autres toxines, et les éliminer sans endommager le foie ;
- cicatriser et soulager les ulcères de l'estomac.

Le document US2004/0081665 décrit l'utilisation d'extraits de chlorelle contenant des polysaccharides de haut poids moléculaire et des complexes de polysaccharides possédant une modulation immunitaire, destinés à augmenter la production de cytokines et utilisés comme composants dans la préparation de vaccins.

Le document KR20030020767 décrit une préparation pharmaceutique, pouvant être sous la forme d'une tablette, d'une capsule ou de granules, pour la prévention et le traitement de l'ostéoporose caractérisée en ce qu'elle contient de la chlorelle comme principe actif.

Le document FR2747922 décrit l'utilisation d'extraits concentrés d'algues du type chlorelle pour élaborer un produit destiné à prévenir et à soigner les maladies de peau, caractérisée en ce que lesdits extraits protègent les cellules de Langerhans de l'épiderme des rayonnements ultraviolets.

Dans la présente invention, le mot algue est employé indépendamment de la taille des algues, donc aussi bien lorsqu'il s'agit de microalgues. De préférence, il s'agit de microalgues.

Aucun document pertinent n'a été identifié par la société Demanderesse concernant l'utilisation d'algues dans la prévention des infections ou maladies bucco-dentaires, et/ou pour l'entretien d'une bonne hygiène bucco-dentaire.

La méthode la plus connue et la plus reconnue pour le maintien d'une bonne hygiène dentaire est un brossage régulier des dents et une utilisation quotidienne du fil dentaire. Cependant ces méthodes ne permettent pas de prévenir les inflammations gingivales et les infections parodontales dues aux problèmes d'inaccessibilité de certaines parties des dents ou des gencives au cours du brossage. Qui plus est, certaines personnes ne souhaitent pas ou n'ont pas la possibilité de se brosser les dents correctement et/ou régulièrement. Par exemple, certaines situations peuvent empêcher un individu d'entretenir une bonne hygiène dentaire, comme la maladie ou l'hospitalisation. D'autres situations rendent le brossage des dents difficile ou impossible : incommodité des lieux, manque d'hygiène, gêne,... Afin d'encourager la santé bucco-dentaire, des essais ont été effectués pour délivrer des principes actifs ou des médicaments au sein de la cavité buccale, comme les antiseptiques, les sels de zinc, les antibiotiques, les oxydants, l'acide folique, la Coenzyme Q10, etc..

Le document WO00/62762 décrit des chewing-gums sans sucre à effet thérapeutique comprenant une gomme de base, une portion soluble, un arôme, du carbonate de calcium et un acide alimentaire, destinés à la reminéralisation de l'émail dentaire et à la prévention et au traitement des caries.

Le document US2007/0110684 décrit une méthode de traitement et de prévention des problèmes dentaires par l'utilisation d'une quantité efficace de *Morinda citrifola,* un extrait d'arbre indien. Les extraits de ce végétal peuvent être sous la forme d'une poudre, d'une tablette, d'un chewing-gum, de granules, d'une pâte dentifrice, d'une lotion pour bains de bouche, en association avec d'autres additifs et/ou principes actifs.

Cependant, l'efficacité, l'absorption, le métabolisme, la biodisponibilité, les effets secondaires, la régulation et les coûts associés à des médicaments et/ou des principes actifs délivrés dans la cavité buccale sont autant de problèmes auxquels il est nécessaire de palier aujourd'hui. Fort de ce constat, la société Demanderesse a eu le mérite de découvrir de façon surprenante et inattendue, que la combinaison des propriétés d'une confiserie, et plus particulièrement d'une confiserie sans sucre, avec celle des microalgues et plus particulièrement la chlorelle, permettait d'obtenir une confiserie non cariogène présentant un effet bénéfique pour la santé bucco-dentaire. En effet, cette confiserie, et plus particulièrement cette confiserie sans sucre, associée à la chlorelle, fournit une solution techniquement et économiquement viable à la prévention des infections ou maladies bucco-dentaires et/ou à l'entretien d'une bonne hygiène bucco-dentaire, permet une utilisation simple, en toutes circonstances et favorise de ce fait le maintien d'une bouche saine tout au long de la journée. Qui plus est, l'utilisation d'une confiserie comme vecteur de la chlorelle permet de délivrer la chlorelle directement sur le lieu des infections buccales, et permet ainsi une optimisation du traitement, à la différence des compléments contenant de la chlorelle existants actuellement et qui doivent être avalés directement. Il est difficile en effet d'imaginer que les extraits de chlorelle contenus dans ces compléments puissent avoir une action bénéfique sur l'hygiène bucco-dentaire une fois arrivés dans l'estomac. D'ailleurs ces compléments ne sont pas reconnus ou prescrits pour la prévention des infections ou maladies bucco-dentaires et/ou l'entretien d'une bonne hygiène bucco-dentaire.

Qui plus est, l'utilisation d'une confiserie comme vecteur permet une utilisation simple, à tout moment de la journée, sans contrainte particulière. L'utilisation d'autres moyens permettant de maintenir une bonne santé bucco-dentaire, i.e. le brossage des dents et/ou l'utilisation de bains de bouche après les repas, présentent en effet le désavantage majeur de ne pas pouvoir être effectué(s) de manière simple, régulière, et à chaque fois que le besoin s'en fait ressentir.

### RESUME DE L'INVENTION

La présente invention concerne donc une confiserie, et plus particulièrement une confiserie sans sucre, contenant des microalgues destinée à la prévention des infections ou maladies bucco-dentaires et/ou à l'entretien d'une bonne hygiène bucco-dentaire, permettant par conséquent le maintien d'une bonne santé bucco-dentaire. La présente invention concerne plus particulièrement des confiseries sans sucre contenant des chlorelles. Enfin, la présente invention concerne également l'utilisation d'une confiserie, de préférence sans sucre, contenant de la chlorelle pour favoriser et maintenir une bonne hygiène, et une bonne santé bucco-dentaires.

### DESCRIPTION DETAILLEE DE L'INVENTION

Dans la présente invention, il faut comprendre par santé bucco-dentaire l'absence de douleur chronique buccale ou faciale, de lésion buccale, de parodontopathie (infection touchant les gencives), de carie et de déchaussement des dents, ainsi que d'autres pathologies et troubles affectant la bouche et la cavité buccale.

Dans la présente invention, le terme infections ou maladies bucco-dentaires désigne les caries, les aphtes, l'halitose, les gingivites, les affections parodontales, et toutes autres lésions buccales.

La carie dentaire est une maladie infectieuse transmissible, qui est le résultat de l'érosion acide générée par les bactéries de la plaque dentaire. Chaque jour, généralement après les repas, la plaque bactérienne se forme rapidement sur les dents et constitue une matrice mince et collante, encore appelée biofilm, à la surface de l'émail des dents et sur les gencives. Cette matrice englobe des débris alimentaires et des bactéries qui se développent grâce aux sucres contenus dans les aliments et les boissons. Les acides produits par les bactéries provoquent une déminéralisation de l'émail qui recouvre les dents. Cette déminéralisation constitue le point de départ de la carie, c'est le stade 1 pour lequel il n'y a pas de sensibilité. Le stade 2 est l'atteinte de la dentine (substance formant la couche interne de la dent) reconnaissable par une sensibilité aux stimuli externes comme le chaud, le froid, le sucre. Le stade 3 se caractérise par une telle destruction des tissus durs qu'il y a atteinte de la pulpe dentaire caractérisable par des douleurs spontanées (rage dentaire). Enfin, le stade 4 est la mortification pulpaire ou dévitalisation spontanée avec une prolifération bactérienne dans les canaux et autour de la dent. C'est le stade le plus grave entrainant une infection ou abcès dentaire.

Ce foyer infectieux représente un danger pour la santé générale. Les bactéries peuvent migrer dans l'organisme par voie sanguine et se greffer sur des organes comme le coeur, les reins, les articulations,... La carie constitue un processus en évolution et il ne peut pas y avoir de guérison spontanée.

Les aphtes et aphtoses bucco-pharyngés occupent une place importante parmi les lésions de la muqueuse buccale. Leur survenue peut être liée à des facteurs nutritionnels, psychologiques (spécialement le stress) ou hygiéniques. Ce sont des ulcères superficiels de la muqueuse. Leur évolution se fait en quatre phases. La phase prodromique est marquée par l'apparition de sensations de brûlures ou de picotement suivie, lors de la phase pré-ulcéreuse, de l'apparition d'une macule érythémateuse centrée sur un point jaune. Celle-ci en se nécrosant va laisser place à une ulcération plus ou moins importante selon la forme clinique caractérisant la phase d'état. La phase cicatricielle conduit à la guérison avec ou sans séquelles selon la taille de la lésion. Lorsque les aphtes buccaux ne sont pas isolés et sont accompagnés d'autres symptômes, ils peuvent être le signe d'une maladie de Behçet ou de maladies inflammatoires de l'intestin comme la colite ulcéreuse ou la maladie de Crohn. Le traitement doit être adapté au degré de sévérité de l'affection et il commence par l'élimination des facteurs locaux favorisants c'est-à-dire par l'élimination du tartre et par une bonne hygiène buccale. Les formes sporadiques relèvent de traitements locaux symptomatiques. Les formes plus sévères nécessitent un traitement systémique dont l'efficacité a été contrôlée. Le traitement idéal devrait agir sur la douleur, sur les lésions elles-mêmes et si possible prévenir les poussées ultérieures.

La fétidité de l'haleine, encore appelée halitose trouve le plus fréquemment ses causes dans la cavité buccale. Le sulfure d'hydrogène et le méthyle mercaptan sont les principaux composants malodorants qui naissent de la dégradation bactérienne des substrats protéiques contenant des groupements thiols et des disulfures qui sont des dérivés primaires des éléments cellulaires dans la salive. La teneur en composés volatils soufrés dans l'haleine est particulièrement élevée en cas d'état inflammatoire ou après de longues périodes de réduction du flux salivaire, pendant le sommeil et après cessation du nettoyage buccal. Cette teneur en composés volatils soufrés dans l'haleine peut être notablement réduite dans la plupart des cas au moyen d'un brossage minutieux de la cavité buccale, y compris la surface dorso-postérieure de la langue ou par un rinçage avec un bain de bouche contenant des sels de zinc. La langue est très souvent la cause de l'halitose. Elle comporte en effet de nombreuses villosités qui retiennent les bactéries responsables de la mauvaise haleine, et forment un dépôt lingual.

La gingivite ou maladie des gencives, se décrit comme l'infection bactérienne des gencives, ou l'inflammation des gencives au stade débutant. Elle se manifeste par des saignements des gencives, mais sans atteinte de l'os qui soutient les dents. Elle peut se traiter par des soins d'hygiène rigoureux à la maison incluant des bains de bouche avec une solution désinfectante, un nettoyage dentaire incluant un détartrage pour éliminer la plaque dentaire et le tartre. Si elle est non-traitée, elle peut conduire à la perte dentaire. La gingivite est la première étape de la parodontite, qui est un état plus agressif de la maladie se décrivant par des saignements des gencives, la destruction de l'os et des tissus soutenant la dent, des dents qui bougent légèrement ou sévèrement, une mauvaise haleine et des poches parodontales (espace profond entre une dent et la gencive). Elle peut se traiter par un curetage et surfaçage qui est une procédure non-chirurgicale de détartrage et de nettoyage en profondeur, par un curetage ouvert qui est une procédure chirurgicale nécessitant l'ouverture de la gencive avant de la cureter, puis la fermeture de celle-ci à l'aide de points de sutures et de pansements. Les stades les plus avancés se traiteront par des greffes osseuses ou des greffes de gencive.

La présente invention concerne une confiserie, et de préférence une confiserie sans sucre à consommer au sein de la cavité buccale, contenant des microalgues pour la prévention des infections ou maladies bucco-dentaires, dont certains exemples ont été détaillés ci-avant, et donc pour l'entretien et le maintien d'une bonne hygiène bucco-dentaire.

Selon un mode préférentiel, lesdites microalgues sont présent (s) à hauteur de 0,2 à 65% en poids, de préférence 0,2 à 50% en poids, de préférence 1 à 40% en poids, de préférence 1 à 30% en poids, et encore de préférence de 5 à 28% en poids ou 10 à 28% en poids.

Selon un mode préférentiel, les microalgues et/ou les extraits de microalgues sont choisis parmi le groupe constitué par *Chlorella, Spirulina* et *Odontella.*

Selon un mode encore plus préférentiel, les microalgues et/ou les extraits de microalgues de la présente invention sont issus du genre *Chlorella,* et de préférence de *Chlorella vulgaris, Chlorella pyrenoidosa, Chlorella regularis, Chlorella sorokiniana,* et de façon encore plus préférentielle *de Chlorella vulgaris.*

Selon un autre mode préférentiel, les confiseries selon la présente invention contiennent des extraits de microalgues, et de préférence des extraits de CGF.

Selon une variante de la présente invention, les microalgues *Chlorella* et/ou ses extraits peuvent être associés avec d'autres algues et/ou d'extraits d'algues possédant également un effet bénéfique sur le bien-être en général.

Des exemples de tels algues et/ou d'extraits d'algues sont par exemple le DHA ou acide docosahexaénoïque, acide gras du groupe des oméga-3 constituant en particulier des cellules nerveuses, contenu dans les algues océaniques des genres *Schizochytrium, Iridaea, Porphyra* et plus particulièrement *Porphyra yezoensis, Gymnogongrus* et plus particulièrement *Gymnogongrus crenulatus et Gymnogongrus griffithsiae, Crypthecodinium* et plus particulièrement *Crypthecodinium cohnii, Hypnéa* et plus particulièrement *Hypnéa musciformis,* Meristothéca et plus particulièrement *Meristothéca senegalensis* et *Meristothéca papulosa.* D'autres exemples d'association avec *Chlorella* et/ou des extraits de *Chlorella* sont les algues des genres *Spirulina, Pheophyceae, Rhodophyceae, Chlorophyceae,* et/ou des extraits de lutéine, zéaxanthine, xanthophylle, laminarine, chlorophylle.

Le terme confiserie (synonymes : bonbons, friandises, sucreries...) désigne dans la présente invention tout produit alimentaire aromatisé présentant une saveur sucrée, dont la consistance peut être dure ou molle, qui peut être enrobée de chocolat et qui se consomme en se suçant et/ou en se mâchant au sein de la cavité buccale.

Selon un mode préférentiel, les confiseries de la présente invention sont toutes les confiseries de type sucres cuits (plus communément appelés bonbons durs), dragées, bonbons gélifiés, gommes, caramels, toffees et fudges, chewing gums, bubble gums, pâtes à mâcher, comprimés, lozenges.

Selon un autre mode préférentiel, les confiseries de la présente invention peuvent être des confiseries aérées comme par exemple les guimauves ou les marshmallows.

Selon un autre mode préférentiel, les confiseries de la présente invention sont préparées avec du chocolat, sous quelque forme que ce soit : tablettes, bonbons, bouchées, truffes, lentilles...

Selon une variante de l'invention, les confiseries de la présente invention peuvent être pelliculées. Le pelliculage consiste en l'application d'une composition liquide filmogène qui devient après séchage un film protecteur. Ce pelliculage sert par exemple à protéger les principes actifs contenus dans la confiserie, à protéger la confiserie elle-même de l'humidité, des chocs, de la friabilité, et également à conférer aux confiseries des propriétés visuelles attractives : brillance, couleur uniforme, surface lisse,...

Selon une variante plus préférentielle, les compositions utilisées pour le pelliculage sont celles décrites dans la demande de brevet WO2005/060944 dont la Demanderesse est titulaire.

Selon un autre mode préférentiel, les confiseries de la présente invention peuvent en plus, quand cela est possible, être fourrées avec des fourrages liquides, pâteux, solides, en poudre.... Elles peuvent également être enrobées de chocolat, dragéifiées, candies, givrées...

Selon un autre mode plus préférentiel, les confiseries de la présente invention sont des confiseries sans sucres. Selon un autre mode encore plus préférentiel, les confiseries de la présente invention sont des confiseries sans sucres, préparées à partir de polyols, de mélange de polyols, de mélange de polyols et de fibres solubles, de mélanges de polyols, de fibres solubles et de protéines, et sont particulièrement adaptées à la présente invention en raison de leur innocuité vis-à-vis des dents et de leur caloricité réduite par rapport au saccharose.

Un exemple d'association particulièrement intéressante est l'utilisation de NUTRIOSE®, qui est une gamme complète de fibres solubles, reconnues pour leurs bienfaits, et fabriquées et commercialisées par la Demanderesse. Le NUTRIOSE® est un dérivé de l'amidon de blé et de maïs partiellement hydrolysé, qui contient jusqu'à 85 % de fibre. Cette richesse en fibre permet d'augmenter la tolérance digestive, d'améliorer la gestion de calorie, de prolonger le dégagement d'énergie et d'obtenir un taux de sucre inférieur. De plus, le NUTRIOSE® est l'une des fibres les mieux tolérées disponibles sur le marché. Il montre une tolérance digestive plus élevée, permettant une meilleure incorporation que d'autres fibres, ce qui représente de vrais avantages alimentaires.

La tendance vers une alimentation plus saine continue à gagner du terrain et modifie les modes de consommation et les habitudes d'achat de façon significative. Consommer moins de sucres tout en continuant à se faire plaisir est le souhait de plus en plus de consommateurs en réponse aux multiples recommandations nutritionnelles.

De plus, les produits sans sucres sont particulièrement intéressants pour la fabrication de confiseries qui ont une fonction thérapeutique, par exemple les bonbons pour la toux ou les voies respiratoires, les chewing-gums contenant du calcium qui contribuent à l'hygiène bucco-dentaire, etc...

Selon un mode encore plus préférentiel de la présente invention, ladite confiserie est un chewing gum sans sucres, à base de polyols pouvant être ou non dragéifié et/ou pelliculé par l'une des méthodes connues et décrites de l'art antérieur.

Sans être limité à un mécanisme particulier, la consommation d'un chewing-gum contenant de la chlorelle est susceptible de contribuer à l'entretien d'une bonne hygiène dentaire, en empêchant entre autre la formation de la plaque dentaire, responsable de beaucoup d'infections ou maladies bucco-dentaires. La mastication dudit chewing gum favorise l'activation des mécanismes de défense de la salive, empêchant ainsi la croissance bactérienne à la surface des dents. L'action combinée de la chlorelle contenue dans le chewing gum renforce encore ces mécanismes de défenses antibactériens.

Cet effet bénéfique se retrouve également lorsque la chlorelle est incluse dans une confiserie à sucer ou à mâcher. A nouveau, la production de salive se trouve favorisée, ce qui permet de réduire considérablement la croissance des bactéries au niveau des dents.

Selon un mode encore plus préférentiel de la présente invention, des arômes tels que la menthe, les arômes de fruits, peuvent être ajoutés pour favoriser une sécrétion salivaire plus importante, et ainsi renforcer l'action de la protection. D'autres arômes tels que par exemple, le menthol, l'eucalyptol, le thymol, le salicylate de méthyl, le réglisse, et l'aldéhyde cinnamique peuvent de par leur propriété antibactérienne inhérente, renforcer l'action de destruction de germes indésirables de la cavité buccale. Selon un autre mode préférentiel de la présente invention, des principes actifs peuvent être ajoutés dans la confiserie. On entend par principe actif, dans la présente invention, toute molécule active possédant un effet pharmacologique démontré et un intérêt thérapeutique également démontré cliniquement.

Selon un autre mode préférentiel de la présente invention, un chewing gum sans sucre contenant de la chlorelle peut être consommé à tout moment afin de prévenir les infections ou maladies bucco-dentaires et permettre l'entretien d'une bonne hygiène bucco-dentaire et par conséquent le maintien d'une bonne santé bucco-dentaire.

Les confiseries selon l'invention peuvent être administrées à des personnes ayant une infection buccale. En particulier, elles conviennent particulièrement à des personnes souffrant d'un manque d'hygiène bucco-dentaire, par choix ou par nécessité. Il est également apprécié que les confiseries de la présente invention puissent tout simplement être utilisées en complément d'une bonne hygiène bucco-dentaire.

Selon un mode préférentiel, une confiserie particulièrement adaptée pourra être un bonbon à texture rugueuse comme celui décrit par la demanderesse dans le brevet EP1222860, permettant à lui tout seul de combattre les problèmes d'halitose. De tels bonbons à texture rugueuse peuvent également être des comprimés, des lozenges, etc. L'action conjointe de la chlorelle et de la texture rugueuse de la confiserie renforce l'action bénéfique au niveau du maintien d'une bouche saine.

Selon un autre mode préférentiel de la présente invention, la quantité de confiserie consommée par jour et par individu se situe entre 0,5 g et 50 grammes. Selon un mode encore plus préférentiel, la quantité consommée par jour se situe entre 2 g et 30 grammes.

Dans le cas particulier où la confiserie est un chewing gum ou une pâte à mâcher, la quantité de confiserie consommée par jour est inférieure à 15 grammes et de préférence de l'ordre de 10 grammes.

Selon un mode préférentiel de la présente invention, la quantité ou dose efficace de chlorelle consommée par jour se situe entre 0,1 g et 10 g. Selon un mode plus préférentiel, la quantité ou dose efficace de chlorelle consommée par jour se situe entre 1 g et 3 g.

Selon un mode encore plus préférentiel, la quantité ou dose efficace de chlorelle et/ou d'extrait de chlorelle consommée par jour est de 2 grammes.

Les confiseries de la présente invention sont consommées à tout moment jugé opportun et durant le temps désiré.

Selon un mode préférentiel, les confiseries sont consommées après chaque ingestion de liquide et/ou de nourriture, comme par exemple après les repas, moments où les attaques bactériennes et la formation de la plaque dentaire sont les plus fréquentes.

Selon un mode préférentiel de la présente invention, les confiseries sont consommées au moins deux fois par jour, et de préférence au moins trois fois par jour.

Selon un mode préférentiel de la présente invention, deux tablettes de chewing-gum sont mâchées conjointement.

Selon un autre mode préférentiel, deux tablettes sont sucées conjointement, et de préférence trois.

Selon un autre mode préférentiel, les confiseries restent présentes dans la cavité buccale pendant au moins 2 minutes, de préférence pendant au moins 5 minutes et encore plus de préférence pendant au moins 10 minutes.

L'invention sera mieux comprise à l'aide des exemples suivants qui se veulent illustratifs mais non limitatifs.

### EXEMPLE 1 : Chewing-gums sans sucre contenant des polyols et de la chlorelle

### A. Formule pour la fabrication des centres (ingrédients exprimés en pourcentage de poids)

- Gomme de base 35,0%
   (Ex : Optima de Cafosa Gum SA Barcelone (Espagne))
- Maltitol Maltisorb® P35 36,3%
- Sirop de maltitol LYCASIN® 80/55 (75% MS) 7,0%
- Chlorelle (poudre 96% MS) 20,0%
- Arôme de menthe liquide 1,5%
   (Ex : Réf SN748096 IFF Company)
- Aspartame 0,2%

### B. Méthode (pour 60 kg de centres)

❖ Procédure de chargement (en minutes) dans un pétrin bras-en-Z à 50°C
   0 min : Introduction dans le pétrin de la gomme de base fondue (chauffée toute une nuit à 50°C) et de la moitié du Maltisorb® P35
   4 min : Ajout de la chlorelle et de l'aspartame
   8 min : Ajout de l'autre moitié du Maltisorb® P35
   10 min : Ajout du Sirop de maltitol LYCASIN® 80/55
   12 min : Ajout de l'arôme de menthe liquide
   14 min : Déchargement du pétrin (gomme à environ 53°C) et division de cette gomme en pains d'environ 2 kilogrammes. Stockage de ces pains pendant environ 1h à un air à 20°C et à 50% humidité relative. La température des pains de gomme est d'environ 43°C lorsqu'ils arrivent à l'extrudeur.
❖ Extrusion (Appareil Togum TO-E82)
   Température du corps de l'extrudeur: 41°C
   Température de la tête de l'extrudeur : 44°C
❖ Laminage : 4 paires de rouleaux - Entaillage : 2 paires de rouleaux (Appareil Togum TO-W191) Saupoudrage des bandes de gomme obtenues avec un mélange contenant 50% en poids de talc et 50% en poids de Mannitol 60.
❖ Stockage : Les bandes de centres non séparés sont stockées pendant environ 48h à un air à 15°C et à 50% humidité relative, avant leur dragéification.

### C. Dragéification des centres obtenus ci-dessus

❖ Composition du sirop de dragéification (70% MS - 75°C)

| | *Ingrédients en poids* | *Composition en MS* |
|---|---|---|
| Maltitol MALTISORB® P200 | 25.00 Kg | 93.25 % |
| Solution de gomme arabique(40 % M.S.) | 3.35 Kg | 5.00 % |
| TiO₂ | 0.27 Kg | 1.00 % |
| Chlorelle | 0.20 Kg | 0.75 % |
| Eau | 9.48 Kg | |
| | | 100.0 % |

❖ Composition de la couche de dragéification (%)
- Maltitol MALTISORB® P200 90,54%
- Solution de gomme arabique(40 % M.S.) 4,85%
- TiO₂ 0,97%
- Arôme de menthe liquide 0,97%
- Chlorelle 0,73%
- Eau 1,94%

❖ Composition du chewing-gum à la chlorelle

| | % | **PAR UNITE g** |
|---|---|---|
| 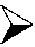 Maltitol MALTISORB® | 55.82 | 1.0184 |
| 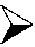 Gomme de base : | 22.40 | 0.4087 |
| 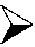 Gomme Arabique | 1.75 | 0.0319 |
| 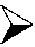 Arôme menthe | 1.31 | 0.0239 |
| 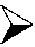 LYCASIN® 80/55 (sec) | 3.36 | 0.0613 |
| 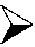 TiO2 | 0.35 | 0.0064 |
| 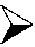 Chlorelle | 13.06 | 0.2383 |
| 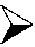 Eau | 1.82 | 0.0332 |
| 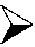 Edulcorant | 0.13 | 0.0024 |

Les chewing-gums ci-dessus contenant de la chlorelle dans la gomme et dans la couche de dragéification périphérique ont été goûtés par un panel de dégustateurs, et leur goût a été jugé satisfaisant et plutôt agréable.

### EXEMPLE 2 : Bonbon à texture rugueuse contenant de la chlorelle

Selon un mode préférentiel, une confiserie particulièrement adaptée pourra être un bonbon à texture rugueuse comme celui décrit par la demanderesse dans le brevet EP1222860.

### A. Formule (ingrédients exprimés en pourcentage de poids)

- Sorbitol NEOSORB® P60W 39,00%
- Maltitol Maltisorb® P200 39,00%
- Chlorelle (poudre 96% MS) 20,00%
- Arôme de menthe liquide 1,00%
   (Ex : Réf SN748096 IFF Company)
- Stéarate de Magnésium 1,00%

### B. Préparation de la poudre

❖ Mélange du Sorbitol et du Maltitol
❖ Pulvérisation de l'arôme liquide sur le mélange poudreux précédent
❖ Ajout de la chlorelle
❖ Ajout du lubrifiant
❖ Mélange jusqu'à homogénéisation

### C. Compression

Compression sur une presse alternative à banc équipée de poinçons plats et ronds de diamètre 18 mm.

### D. Caractéristiques des comprimés obtenus

❖ Poids par comprimé : 1,76 g
❖ Epaisseur : 5 mm
❖ Dose de chlorelle par comprimé : 352 mg
❖ Dureté mesurée sur l'appareil Erweka TBH 30 : 110 N Les bonbons rugueux ont été goûtés par un panel de dégustateurs, et leur goût a été jugé satisfaisant et plutôt agréable.

### EXEMPLE 3 : Recettes de pâtes à mâcher contenant de la chlorelle

### A. Formule pour les deux essais

| | Ingrédients en poids (g) | |
|---|---|---|
| | Essai 1 | Essai 2 |
| Sirop de maltitol LYCASIN® HBC (73% MS) | 584 | 710 |
| Mannitol 60 | 126 | 0 |
| Graisse végétale* | 40 | 40 |
| Monostéarate de Glycérol | 3 | 3 |
| Gélatine 125blooms (solution à 40% MS) | 17 | 17 |
| Chlorelle(96% MS) | 230 | 230 |
| Arôme pomme** | 0 | qsp(1,5ml) |
| Total | 1000g | 1000g |
| Température de cuisson | 116°C | 116°C |
| aw | 0,54 | 0,5 |

| | | |
|---|---|---|
| * Biscuitine 621 de Loders Croklaan ** Réf SN748106 IFF Company | | |

### B. Méthode

❖ Préparation de la solution de gélatine à 40% de M.S.
❖ Cuisson du LYCASIN® HBC (et du Mannitol 60, pour l'essai 1) à 116°C.
❖ Dans un mixer, ajout de la solution de gélatine et malaxage.
❖ Ajout de la graisse végétale fondue et de l'émulsifiant, et malaxage.
❖ Ajout de la chlorelle et de l'arôme, et malaxage.
❖ Refroidissement, découpage et emballage.

### C. Composition des produits finis des deux essais

| | Composition des produits(%) | |
|---|---|---|
| | Essai 1 | Essai 2 |
| LYCASIN® HBC (73% MS) | 46,6 | 59,1 |
| Mannitol 60 | 13,8 | 0,0 |
| Graisse végétale | 4,4 | 4,6 |
| Monostéarate de Glycérol | 0,3 | 0,3 |
| Gélatine 125blooms (40% solution) | 0, 7 | 0, 8 |
| Chlorelle | 24,2 | 25,2 |
| Arôme pomme | qs | qs |
| Eau résiduelle | 10,0 | 10,0 |
| Total | 100,0 | 100,0 |

Les pâtes à mâcher à l'arôme de pomme ont été goutées par un panel de dégustateurs, et leur goût a été jugé satisfaisant et plutôt agréable.

### EXEMPLE 4 : Confiserie gélifiée à la gélatine et fourrée à la chlorelle

A. Formule (ingrédients exprimés en pourcentage de poids)

| | Ingrédients en poids (g) | |
|---|---|---|
| | Préparation A | Préparation B |
| Sucrose | 311 | 105 |
| Sirop de glucose (42 DE) (75% DS) | 408 | 231 |
| Gélatine (220Blooms) (40%MS) | 170 | / |
| Acide citrique (50% MS) | 17 | 18 |
| Arôme et colorant | Qs | 17 |
| Eau | 94 | 389 |
| Amidon CLEARAM® CR2010 | / | 46 |
| Chlorelle | / | 194 |
| Total | 1000g | 1000g |

### B. Méthode

❖ Préparation et cuisson de la masse extérieure gélifiée à la gélatine (Préparation A)
   - Placer la gélatine dans un bain-marie à 60°C.
   - Mélanger le sucre avec le sirop de glucose et l'eau, et commencer à faire chauffer l'ensemble.
   - Cuire le mélange précédent à 110°C - 115°C.
   - Refroidir l'ensemble, et à 90°C ajouter la solution de gélatine, l'arôme acide citrique et le colorant. Le Brix du mélange est d'environ 78%.
❖ Préparation et cuisson du fourrage à la chlorelle (Préparation B)
   - Mélanger le sucre avec le sirop de glucose et l'eau, et commencer à faire chauffer l'ensemble.
   - Disperser l'amidon CLEARAM® CR2010 dans de l'eau froide, ajouter l'ensemble au mélange précédent et faire cuire pour gélatiniser le tout et obtenir une masse transparente.
   - Disperser la chlorelle dans l'eau.
   - Ajouter la solution de chlorelle à la masse transparente précédemment obtenue.
❖ Réalisation de ladite confiserie
   - Déposer la masse extérieure gélifiée (préparation A) et le fourrage à la chlorelle (préparation B) dans une couleuse comportant deux trémies de coulée pour produits fourrés.
   - Régler la couleuse pour que le produit fini comporte 90% de la masse (préparation A) et 10% de fourrage (préparation B).
   - Déposer les confiseries fourrées dans de l'amidon de coffrage sec et laisser reposer pendant 24 heures.
   - Les retirer et les brosser avant de les huiler.

### C. Composition du produit fini

| | Composition des produits en % |
|---|---|
| Sucrose | 34,45 |
| Sirop de glucose (42 DE) (75% DS) | 34,73 |
| Gélatine (220Blooms) (40%MS) | 7,20 |
| Acide citrique (50% MS) | 1,08 |
| Arôme et colorant | qs |
| Eau résiduelle | 20,10 |
| Amidon CLEARAM® CR2010 | 0,5 |
| Chlorelle | 1,94 |
| Total | 100% |

Les confiseries gélifiées à la gélatine et fourrées à la chlorelle ont été goutées par un panel de dégustateurs, et leur goût a été jugé satisfaisant et agréable.

### EXEMPLE 5 : Comprimés contenant de la chlorelle

### A. Formule (ingrédients exprimés en pourcentage de poids)

- LYCATAB® C 37,25%
- Chlorelle (poudre 96% MS) 62,50%
- Stéarate de Magnésium 0,25%

### B. Préparation de la poudre

Mélange des trois ingrédients entre eux à la main pendant 5 minutes.

### C. Compression

Compression sur une presse rotative FETTE P 1000 équipée de poinçons ronds concaves de diamètre 16 mm et de rayon de courbure de 25 mm.

### D. Caractéristiques des comprimés obtenus

- Poids par comprimé : 1000 mg
- Epaisseur : 5,76 mm
- Dose de chlorelle par comprimé : 625 mg
- Dureté mesurée sur l'appareil Erweka TBH 30 : 40 N

Les comprimés contenant de la chlorelle ont été goûtés par un panel de dégustateurs, et leur goût a été jugé satisfaisant et plutôt agréable.

### EXEMPLE 6 : Comprimés contenant de la chlorelle et du NUTRIOSE®

### A. Formule (ingrédients exprimés en pourcentage de poids)

- NUTRIOSE® FB06 49,75%
- Chlorelle (poudre 96% MS) 49,75%
- Stéarate de Magnésium 0,50%

### B. Préparation de la poudre

Mélange des trois ingrédients entre eux.

### C. Compression

Compression sur une presse rotative FETTE P 1000 équipée de poinçons ronds concaves de diamètre 16 mm et de rayon de courbure de 25 mm.

### E. Caractéristiques des comprimés obtenus

- Poids par comprimé : 1000 mg
- Epaisseur : 5,75 mm
- Dose de chlorelle par comprimé : 497 mg
- Dureté mesurée sur l'appareil Erweka TBH 30 : 40 N

Les comprimés contenant de la chlorelle en association avec du NUTRIOSE® ont été goûtés par un panel de dégustateurs, et leur goût a été jugé satisfaisant et plutôt agréable.

### EXEMPLE 7 Effet de la consommation de confiseries à la chlorelle sur la diminution de la plaque dentaire

Une étude en interne a été réalisée afin de mesurer l'efficacité de la consommation des confiseries décrites dans l'exemple 2 de la présente demande, sur la réduction de la formation de plaque dentaire, à l'origine de bien des infections ou maladies bucco-dentaires.

La plaque dentaire est définie comme une accumulation hétérogène, adhérente à la surface des dents ou logée dans l'espace gingivo-dentaire, composée d'une communauté microbienne riche en bactéries aérobies et anaérobies qui sont enrobées dans une matrice intercellulaire d'origine microbienne et salivaire. C'est un gel blanc jaunâtre, terne, tenace, semblable à de la colle. La communauté microbienne variée, localisée à la surface de la dent se développe là où persistent les restes alimentaires en les utilisant pour leur métabolisme. La plaque dentaire déséquilibre l'écosystème buccal et favorise l'installation de pathologies buccales. Elle est responsable de l'agression carieuse. Elle est aussi considérée comme le point de départ de la maladie parodontale (gingivite, parodontite). Elle peut également avoir un rôle dans l'halitose.

### Protocole expérimental

15 volontaires ont été choisis, parmi lesquels se trouvent :
- 8 femmes dont l'âge est compris entre 20 et 55 ans ;
- 7 hommes dont l'âge est compris entre 20 et 55 ans ;

Dont :
- 5 fumeurs ;
- 10 consommateurs d'au moins 3 cafés ou thés par jour.

Ces volontaires ne souffrent d'aucun problème dentaire particulier et ont une bonne hygiène bucco-dentaire : brossage des dents deux fois par jour (le matin et le soir). Le test a consisté à mesurer la plaque dentaire 1 heure après la prise du déjeuner, grâce à un révélateur coloré de plaque dentaire. Ce révélateur se présente sous la forme d'un liquide ou de comprimés, et est disponible en pharmacie. Le produit contient de l'érythrocine qui colore en rose fuchsia la plaque dentaire sur les dents, et permet ainsi de la révéler afin de pouvoir visuellement la quantifier.

La quantification de la plaque dentaire a été faite visuellement en appréciant la surface dentaire colorée par rapport à la surface dentaire totale.

Durant les dix premiers jours, les volontaires n'ont pas consommé les confiseries de la présente invention, et n'ont pas suivi de restriction alimentaire ou tabagique. Leur plaque dentaire a été révélée 1 heure après la fin de la prise du déjeuner.

Puis, pendant les dix jours suivant, les volontaires ont consommé deux tablettes rugueuses décrites dans l'exemple 2, en les laissant fondre tout en les faisant circuler dans la cavité buccale pendant au moins 5 minutes, une demi-heure après avoir pris leur déjeuner. Puis une heure après la fin de leur déjeuner, soit environ 20 minutes après la fonte complète des confiseries, leur plaque a été révélée dans les mêmes conditions, en utilisant un révélateur liquide. A nouveau, les volontaires n'ont pas suivi de restriction alimentaire ou tabagique.

### Résultats des mesures

- Lorsque les volontaires n'ont pas consommé les confiseries de la présente invention, la plaque dentaire révélée 1h après la fin du déjeuner a été évaluée pour 13 des 15 volontaires comme représentant plus de 60% de la surface dentaire totale visible. Pour les deux volontaires restants, la plaque dentaire formée 1h après la fin du déjeuner a été évaluée visuellement comme représentant 40% de la surface dentaire totale visible.
- Lorsque les volontaires ont consommé les confiseries de la présente invention selon le protocole expérimental, la plaque dentaire révélée a été évaluée comme représentant seulement 20% de la surface dentaire totale visible, pour 12 des 15 volontaires. La plaque dentaire des 3 volontaires restant a été évaluée comme représentant 30% de la surface dentaire totale visible.

Cet exemple démontre que la consommation régulière des confiseries de la présente invention permet une réduction significative de la formation de plaque dentaire, et participe de ce fait au maintien d'une bonne hygiène bucco-dentaire. Qui plus est, la consommation des confiseries selon l'invention permet de remplacer le brossage des dents après le déjeuner qui est souvent écarté pour des raisons de facilité ou de gêne.

## Revendications

1. Confiserie contenant des microalgues issues du genre Chlorella pour son utilisation dans la prévention des infections ou maladies bucco-dentaires, et dans l'entretien d'une bonne hygiène bucco-dentaire.

2. Confiserie selon la revendication 1 pour son utilisation dans la prévention des infections ou maladies bucco-dentaires, contenant 0,2 à 65% en poids, et de préférence 1 à 40%, et encore de préférence de 5 à 28% de microalgues.

3. Confiserie selon l'une quelconque des revendications 1 à 2 pour son utilisation dans la prévention des infections ou maladies bucco-dentaires, **caractérisée en ce qu'**elle ne contient pas de sucres.

4. Confiserie selon la revendication précédente pour son utilisation dans la prévention des infections ou maladies bucco-dentaires, **caractérisé en ce qu'**elle contient un ou plusieurs polyols, ou un ou plusieurs polyols associé(s) à une ou plusieurs fibres solubles, ou un ou plusieurs polyols associé(s) à une ou plusieurs fibres solubles et à une ou plusieurs protéines.

5. Confiserie selon l'une quelconque des revendications 1 à 4 pour son utilisation dans la prévention des infections ou maladies bucco-dentaires, dans laquelle les microalgues sont issues de *Chlorella vulgaris, Chlorella pyrenoidosa, Chlorella regularis,* ou *Chlorella sorokiniana.*

6. Confiserie selon la revendication 5 pour son utilisation dans la prévention des infections ou maladies bucco-dentaires, dans laquelle les microalgues sont associés avec d'autres algues et/ou extraits d'algues choisi dans le groupe constitué par les algues des genres *Schizochytrium, Iridaea, Porphyra, Gymnogongrus, Crypthecodinium, Hypnéa, Meristothéca, Spirulina, Pheophyceae, Rhodophyceae, Chlorophyceae,* les extraits de lutéine, zéaxanthine, xanthophylle, laminarine, chlorophylle, et l'acide docosahexaénoïque.

7. Confiserie selon l'une quelconque des revendications 1 à 6 pour son utilisation dans la prévention des infections ou maladies bucco-dentaires, **caractérisé en ce qu'**elle contient également un principe actif.

8. Confiserie selon l'une quelconque des revendications 1 à 7 pour son utilisation dans la prévention des infections ou maladies bucco-dentaires, dans laquelle la confiserie est sélectionnée dans le groupe constituée par les sucres cuits, les dragées, les bonbons gélifiés, les gommes, les caramels, les toffees et fudges, les pâtes à mâcher, les comprimés, les guimauves, les marshmallows, le chocolat, les chewing-gums, les bubble gums, les lozenges.

9. Confiserie selon l'une quelconque des revendications 1 à 8 pour son utilisation dans la prévention des infections ou maladies bucco-dentaires, dans laquelle la confiserie est fourrée avec un fourrage liquide, pâteux, solide, et/ou en poudre.

10. Utilisation de chlorelle pour la fabrication d'une confiserie destinée à la prévention des infections ou maladies bucco-dentaires, et à l'entretien d'une bonne hygiène bucco-dentaire.

## Patentansprüche

1. Süßigkeit, welche von der Gattung Chlorella stammende Mikroalgen zur Verwendung in der Prävention von Infektionen oder Erkrankungen des Mund- und Zahn-Bereichs und zur Gewährleistung einer guten Mund- und Zahnhygiene umfasst.

2. Süßigkeit nach Anspruch 1 zur Verwendung in der Prävention von Infektionen oder Erkrankungen des Mund- und Zahn-Bereichs, welche 0,2 bis 65 Gewichtsprozent, und bevorzugt 1 bis 40% und noch weiter bevorzugt 5 bis 28% Mikroalgen umfasst.

3. Süßigkeit nach einem der Ansprüche 1 bis 2 zur Verwendung in der Prävention von Infektionen oder Erkrankungen des Mund- und Zahn-Bereichs, **dadurch gekennzeichnet, dass** sie keine Zucker enthält.

4. Süßigkeit nach dem vorhergehenden Anspruch zur Verwendung in der Prävention von Infektionen oder Erkrankungen des Mund- und Zahn-Bereichs, **dadurch gekennzeichnet, dass** sie ein oder mehrere Polyole oder ein oder mehrere Polyole in Verbindung mit einer oder mehreren löslichen Fasern oder ein oder mehrere Polyole in Verbindung mit einer oder mehreren löslichen Fasern und einem oder mehreren Proteinen enthält.

5. Süßigkeit nach einem der Ansprüche 1 bis 4 zur Verwendung in der Prävention von Infektionen oder Erkrankungen des Mund- und Zahn-Bereichs, wobei die Mikroalgen von *Chlorella vulgaris, Chlorella pyrenoidosa, Chlorella regularis* oder *Chlorella sorokiniana* stammen.

6. Süßigkeit nach Anspruch 5 zur Verwendung in der Prävention von Infektionen oder Erkrankungen des Mund- und Zahn-Bereichs, wobei die Mikroalgen mit anderen Algen und/oder Algenextrakten verbunden sind, die gewählt sind aus der Gruppe, umfassend Algen der Gattungen *Schizochytrium, Iridaea, Porphyra, Gymnogongrus, Crypthecodinium, Hypnea, Meristotheca, Spirulina, Pheophyceae, Rhodophyceae, Chlorophyceae,* Luteinextrakte, Zeaxanthin, Xantophyll, Laminarin, Chlorophyll und Docosahexaensäure.

7. Süßigkeit nach einem der Ansprüche 1 bis 6 zur Verwendung in der Prävention von Infektionen oder Erkrankungen des Mund- und Zahn-Bereichs, **dadurch gekennzeichnet, dass** sie ferner einen Wirkstoff enthält.

8. Süßigkeit nach einem der Ansprüche 1 bis 7 zur Verwendung in der Prävention von Infektionen oder Erkrankungen des Mund- und Zahn-Bereichs, wobei die Süßigkeit gewählt ist aus der Gruppe, umfassend gekochte Zucker, Dragees, Gelee, Gummi, Karamell, Toffee und Fudge, Kaubonbons, Tabletten, Schaum, Marshmallows, Schokolade, Chewing-Gums, Bubble-Gums, Lutschtabletten.

9. Süßigkeit nach einem der Ansprüche 1 bis 8 zur Verwendung in der Prävention von Infektionen oder Erkrankungen des Mund- und Zahn-Bereichs, wobei die Süßigkeit mit einer flüssigen, pastösen, festen und/oder pulverförmigen Füllung gefüllt ist.

10. Verwendung von Chlorella zur Herstellung einer Süßigkeit zur Verwendung in der Prävention von Infektionen oder Erkrankungen des Mund- und Zahn-Bereichs und zur Gewährleistung einer guten Mund- und Zahnhygiene.

## Claims

1. A confectionery containing microalgae derived from *Chlorella* for its use in the prevention of oral-dental infections or diseases and for the upkeep of good oral-dental hygiene.

2. The confectionery as claimed in claim 1 for its use in the prevention of oral-dental infections, containing 0.2 to 65% by weight, and preferably 1 to 40%, and more preferably from 5 to 28% of microalgae.

3. The confectionery as claimed in either one of claims 1 and 2 for its use in the prevention of oral-dental infections, **characterized in that** it is sugar-free.

4. The confectionery as claimed in the preceding claim for its use in the prevention of oral-dental infections, **characterized in that** it contains one or more polyols, or one or more polyols combined with one or more soluble fibers, or one or more polyols combined with one or more soluble fibers and one or more proteins.

5. The confectionery as claimed in anyone of claims 1 to 4 for its use in the prevention of oral-dental infections, in which the microalgae are derived from *Chlorella vulgaris, Chlorella pyrenoidosa, Chlorella regularis, Chlorella sorokiniana.*

6. The confectionery as claimed in claim 5 for its use in the prevention of oral-dental infections, in which the microalgae are combined with other algae and/or extracts of algae chosen from the group constituted by the algae of the genera *Schizochytrium, Iridaea, Porphyra, Gymnogongrus, Crypthecodinium, Hypnea, Meristotheca, Spirulina, Pheophyceae, Rhodophyceae, Chlorophyceae,* the extracts of lutein, zeaxanthin, xanthophyll, laminarin, chlorophyll and docosahexaenoic acid.

7. The confectionery as claimed in any one of claims 1 to 6 for its use in the prevention of oral-dental infections, **characterized in that** it also contains an active principle.

8. The confectionery as claimed in any one of claims 1 to 7 for its use in the prevention of oral-dental infections, in which the confectionery is selected from the group constituted by boiled sugars, dragees, jelly candies, gums, caramels, toffees and fudges, chewy pastes, tablets, marshmallows, chocolate, chewing gums, bubble gums and lozenges.

9. The confectionery as claimed in any one of claims 1 to 8 for its use in the prevention of oral-dental infections, in which the confectionery is filled with a liquid, pasty, solid and/or powdered filling.

10. The use of chlorella for the manufacture of a confectionery intended for the prevention of oral-dental infections or diseases and for the upkeep of good oral-dental hygiene.
